# EUROPÄISCHE PATENTSCHRIFT

(11) **EP 2 181 731 B1**
(45) Veröffentlichungstag und Bekanntmachung des Hinweises auf die Patenterteilung: **28.12.2011**
(21) Anmeldenummer: 09012354.8
(22) Anmeldetag: 15.01.2004
(51) Int. Cl.: A61P 7/02, A61K 38/00

(54) **Organische Verbindungen mit biologischer Wirkung als Thrombinhemmer und ihre Verwendung**
Organic compounds with biological effect as thrombin inhibitor and use of same
Liaisons organiques dotées d'un effet biologique comme inhibiteurs de thrombine et leur utilisation

(30) Priorität: 15.01.2003 DE 10301255
(43) Veröffentlichungstag der Anmeldung: 05.05.2010
(62) Teilanmeldung aus: 04702312.2
(73) Patentinhaber: CPI Creative Pharma International GmbH, 37085 Göttingen (DE)
(72) Erfinder: Thürk, Marcel, 37120 Bovenden (DE); Schwienhorst, Andreas, 18233 Teßmannsdorf (DE)
(74) Vertreter: Held, Stephan

(56) Entgegenhaltungen:
- WO-A-03/022873
- CLAESON G: "SYNTHETIC PEPTIDES AND PEPTIDOMIMETICS AS SUBSTRATES AND INHIBITORSOF THROMBIN AND OTHER PROTEASES IN THE BLOOD COAGULATION SYSTEM" BLOOD COAGULATION & FIBRINOLYSIS, RAPID COMMUNICATIONS, OXFORD,OXFORD, GB, Bd. 5, 1994, Seiten 411-436, XP000918308 ISSN: 0957-5235
- KAMPHAUSEN STEFAN ET AL: "Genetic algorithm for the design of molecules with desired properties." JOURNAL OF COMPUTER-AIDED MOLECULAR DESIGN, Bd. 16, Nr. 8-9, 2002, Seiten 551-567, XP002295150 ISSN: 0920-654X
- ELG M ET AL: "EFFECTS OF AGENTS, USED TO TREAT BLEEDING DISORDERS, ON BLEEDING TIME PROLONGED BY A VERY HIGH DOSE OF A DIRECT THROMBIN INHIBITOR IN ANESTHESIZED RATS AND RABBITS" THROMBOSIS RESEARCH, TARRYTOWN, NY, US, Bd. 101, 1. Januar 2001 (2001-01-01), Seiten 159-170, XP002948831 ISSN: 0049-3848
- RIESTER DANIEL ET AL: "Thrombin inhibitors identified by computer-assisted multiparameter design" PROCEEDINGS OF THE NATIONAL ACADEMY OF SCIENCES OF THE UNITED STATES OF AMERICA, Bd. 102, Nr. 24, Juni 2005 (2005-06), Seiten 8597-8602, XP002571000 ISSN: 0027-8424

## Beschreibung

Diese Erfindung betrifft biologische Wirkstoffe, die mit Thrombin interagieren und dieses hemmen. Die Wirkstoffe sind als Antikoagulationsmittel für Menschen und Tiere nützlich. Insbesondere erstreckt sich diese Erfindung auf kleine peptidische Moleküle der Länge 3-6 Aminosäuren der allgemeinen Formel (I)

Y¹-(NH-X¹-C=O) -(NH-X²-C=O)-(NH-X³-C=O)-(NH-X⁴-C=O)-(NH-X⁵-C=O)-(NH-X⁶-C=O)-Y² (I)

in der Y¹, Y² und X¹⁻⁶ die in den Ansprüchen 1 und 2 genannten Bedeutungen haben. Die Erfindung betrifft auch Zusammenstellungen und Kombinationen mit diesen Stoffen für therapeutische, prophylaktische und diagnostische Zwecke.

Akut Gefäßerkrankungen, wie der myokordiale Infarkt, der Schlaganfall, die Lungenembolie, die tiefe Venenthrombose oder die periphere Gefäβverstopfung und andere Thrombosen des Blutsystems, bilden eine wesentliche gesundheitliche Gefahrenquelle. Derartige Erkrankungen werden durch völlständige oder teilweise Verstopfung des Blutgefäßes durch einen Pfropfen, der Fibrin und Blutplättchen enthält, hervorgerufen.

Gegenwärtige Methoden der Behandlung und Prophylaxe solcher Thromboseerkrankungen umfassen Therapeutika, die auf zwei verschiedenen Wegen wirken. Der erste Typ der Therapeutika verhindert die Thrombin-Aktivität oder Thrombose-Bildung und damit die Bildung des Pfropfens. Diese Medikamente verhindern auch die Entwicklung von Blutplättchen und deren Aggregation. Die zweite Kategorie von Medikamenten beschleunigt die Thrombolyse und löst den Pfropfen auf, beseitigt damit diesen aus dem Blutgefäß und hebt die Blockade des Blutflusses auf.

Heparin und Cumarin, Präparate des ersten Typs, werden in großem Maße zur Behandlung von venöser Thromboseembolien eingesetzt, in der die thrombotische Aktivität für die Entwicklung und Ausdehnung des Thrombus verantwortlich ist. Obgleich wirkungsvoll, ruft Heparin jedoch viele unerwünschte Nebenwirkungen hervor, wie Blutungen oder Thrombocytopenie. Das gleiche gilt für Cumarin, das durch die Blockade oder Verhinderung der Bildung von Prothrombin wirkt und einige Zeit bis zu seiner vollen Wirkungsentfaltung benötigt. Zusammengenommen hat dies zu einer Suche nach spezifischer wirkenden und weniger toxischen Antigerinnungsmitteln geführt, wie z.B. peptidischen Hemmern.

Hirudin ist ein natürliches vorkommendes Polypeptid, das vom Blutegel *Hirudo medicinalis* produziert wird. Dieser wirkstoff, der in der Speicheldrüse des Blutegels synthetisiert wird, ist der wirkungsvollste bekannte natürliche Gerinnungshemmer. Hirudin ist ein direkter Thrombininhibitor und unterbindet die Koagulation des Blutes durch eine starke Bindung an das Thrombin (Kd=2x10⁻¹⁴ M) in einem stöchiometrischen 1 : 1 Komplex [Stone & Hofstenge, Kinetics of the inhibition of thrombin by hirudin, Biochemistry 25, S. 4622-4628 (1986)]. Dieses wiederum verhindert, daß das Thrombin die Umwandlung von Fibrinogen zu Fibrin (dem Pfropfen) katalysiert, wie es auch alle anderen Thrombin-venmittelten Spaltungsprozesse verhindert.

In Tierstudien wurde die Effizienz von Hirudin, in gereinigter Form aus Blutegeln gewonnen, bei der Prävention von venöser Thrombose, Gefäßverstopfung sind Thrombininduzierter verbreiteter intravaskulärer Koagulation demonstriert. Darüber hinaus zeigt Hirudin eine geringe Toxizität, geringe Antikörperbildung und eine schnelle Abbaubarkeit aus dem Blutkreislauf [F.Markwardt et al., Pharmacological studies on the antithrombic action of Hirudin in experimental animals, Thromb. Haemost. 47, S. 226-229 (1982)]. In Projekten, die darauf abzielen, größere Mengen von Hirudin herzustellen, wurden Versuche unternommen, das Polypeptid durch rekombinante DNA-Technologie herzustellen. Die Gegenwart eines O-sulfatisierten Tyrosin-Rests im natürlichen Hirudin und die Unfähigkeit der Mikroorganismen, eine gleichartige Modifikation durchzuführen, machten die Aussicht auf eine rekombinante Produktion biologisch aktive Hirudins hochspekulativ. Die Beobachtung, daß desulfatisiertes Hirudin fast so wirkungsvoll ist wie das sulfierte Gegenstück [U.S.Pat.No. 4 654 302], zeigte den Weg zur Klonierung und Darstellung in verschiedenen Expressionssystemen auf, unter anderem *S. cerevisiae* [Harvey et al., Cloning and expression of cDNA coding for the anticoagulant hirudin from the bloodsucking leech, Hirudo medicinalis, PNAS 83, S. 1084-1088; Europ.Pat.Appl. 158 654, 168 342 und 171 024], *E. coli* [Bergmann et al., Chemical synthesis and expression ob a gene coding for hirudin, the thrombin-specific inhibitor from the leech Hirudo medicinalis, Biol. Chem. Hoppe-Seyler 367, S. 731-740; Europ.Pat.Appl. 200 655] und auf den Spitzen eines filamentösen Phagen als Verschmelzungprotein mit Protein III (pIII). [Wirsching et al., Display of functional thrombin inhibitor hirudin on the surface of phage M13, Gene 204, S. 177-184]. Trotz dieser Fortschritte ist Hirudin in der Produktion nach wie vor recht teuer. Dennoch hat es die dritte klinische Phase durchlaufen und wurde kürzlich für die Behandlung von Heparin-induzierter Thrombocytopenia zugelassen (HMR).

Erst kürzlich wurden bei der Identifikation von Peptidfragmenten natürlichen Hirudins Erfolge erzielt, die ebenso die Koagulationszeit verlängern. Solche Peptidfragmente können jedoch aufgrund ihrer geringen Wirksamkeit bezüglich der Verhinderung der Pfropfenbildung nicht voll zufriedenstellen. So hat z.B. N-Acetyl-Hirudin₄₅₋₆₅ eine um vier Größenordnungen geringere Wirksamkeit als natürliches Hirudin, obgleich es nach wie vor ein relativ großes Molekül ist. Das Problem eher geringer Affinitäten für Thrombin wurde durch die Entwicklung von Hirulogen [U.S.Pat.No. 5433940] gelöst. Diese Moleküle ahmen die Wirkung von Hirudin dadurch nach, daß sie sowohl an die nach außen gerichtete Anion-bindende Seite geringer Affinität binden, wie auch an die katalytische Seite des α-Thrombins. Von daher sind Hiruloge durch eine an die Anion-bindende Thrombin-Außenseite assoziierte Hälfte, eine Verbindungsgruppe und einen gegen das katalytische Zentrum des Thrombins gerichteten Anteil charakterisiert. Das am meisten bevorzugte Hirulog ist Hirulog-8, ein Peptid aus 20 Aminosäuren, das aus dem das katalytische Zentrum hemmenden Peptid D-Phe-Pro-Arg-Pro-, einer Gly₄₋Verbindungs-Sequenz und der Sequenz -Asn-Gly-Asp-Phe-Glu-Glu-Ile-Pro-Glu-Glu-Tyr-Leu-OH des Hirudins aufgebaut ist. Hirulog-8 befindet sich seit kurzem in den USA auf dem Markt.

Trotz der Fortschritte relativ hoher Wirksamkeit für Thrombin (Kᵢ = 2,3nM) sind Hiruloge immer noch relativ große Moleküle, die in relativ mühsame Schemata, wie gemischten heterologen / festen Phasen, synthetisiert werden müssen. Wie Hirudin sind Hirologe nur parenteral hilfreich und müssen sorgfältig überwacht werden. Daher sind Hiruloge nicht als Leitstrukturen für kleine Molekül geeignet, die schließlich auch oral verabreicht werden könnten.

Daher gab es einige Anstrengungen, um kleinere Peptide als potente Thrombinhemmer zu identifizieren. Bereits 1956 zeigte Bettelheim, daß das Fibrinopeptid A die Reaktion zwischen Thrombin und Fibrinogen vergleichbar hemmt. In einer gemeinsamen Forschung von Blombäck und der Nobel Pharma/Kabi in Stockholm wurden vom Fibrinopeptid A abgeleitete Peptidsequenzen mit nicht mehr als neun Aminosäuren mit guter Wirksamkeit auf Thrombin gefunden. Wesentliche Bestandteile der Wirksamkeit waren ein N-endständiges Phe und ein C-endständiges Arg, getrennt durch sieben Aminosäuren. Weniger Aminosäuren verringerten die Wirksamkeit, aber erstaunlicherweise zeigte ein Tripeptid mit N- und C-endständigem Phe bzw. Arg exzellente Wirksamkeit. Das beste Tripeptid mit hemmender Wirkung auf die Thrombin-Fibrinogen-Reaktion war Bz-Phe-Val-Arg-OMe, wobei Val wie im Fibrinopeptid ganzer Länge dem Arg vorausgeht [Blombäck et al., Synthetic peptides with anticoagulant and vaodilating activity, Scand. J. Clin. Lab. Invest. 24, S. 59-66 (1969), U.S.Pat.No. 3 826 794 (1974)]. Im Gegensatz zum Fibrinopeptid A geht Pro dem Arg in einer Reihe anderer Thrombinausschnittsbereiche, wie der des Prothrombins, des Faktors XIII und der menschlichen Wachstumshormone voraus. Auf Basis der Pro-Arg-Sequenz wurden die meisten der heute wirksamsten Thrombin-hemmenden Peptide und Peptidomimetiken entwickelt. Unter diesen wirkungsvollsten Hemmern befindet sich H-D-Phe-Pro-Arg-H (K*ᵢ* = 70 nM) [Bajuz et al., Inhibition of thrombin and trypsin by tripeptide aldehydes, Int. J. Peptide Protein Res. 12, S. 217-221 (1978); Hung.Pat. 169 870 (1974)]. Die Idee zu diesem Peptidaldehyd erwuchs aus der Entdeckung von Peptidaldehyden bakteriellen Ursprungs durch H. Umezawa. Diese so genannten Leupeptide (z.B. Ac-Leu-Leu-Arg-H) sind Hemmer des Plasmins und anderer Trypsin-ähnlicher Proteasen, jedoch nicht des Thrombins. Der Aldehyd-Kohlenstoff hat in seiner acetalen Form eine tetraedrale Struktur, die gleiche wie der Carbonyl-Kohlenstoff von Substraten in der Übergangsphase.

Aus diesen eben erwähnten Aldehyden synthetisierten Shaw et al. den irreversiblen Chlormethylketon-Hemmer H-D-Phe-Pro-Arg-CH₂-Cl mit einem K*ᵢ* von 25 nM [Kettner et al., H-D-Phe-Pro-Arg-CH2-Cl - a selective activity label for thrombin, Thromb. Res. 14, S.969-973 (1979)]. Entwicklungsarbeiten bei Eli Lilly führten zu N-Methyl-D-Phe-Pro- Arg-H, auch als Efegatran [tm] bekannt. Die D-Phe-Pro-Arg-Sequenz wurde kürzlich nochmals weiterentwickelt. Spekulationen, daß eine N-endständige Aminosäure mit aromatischen / lipophilen Gruppen eine größere Wirksamkeit gegenüber Thrombin ergeben könnte, führten zur Entdeckung einiger Hemmer mit neuen Aminosäuren an dieser Position, darunter das β - β-Diphenylalanin (Dpa), Phenylglycin, Cyclohexylglycin, Carboxy-1,2,3,4-tetrahydroisoquinolin (Tiq) [Schuman et al., Highly selective thrombin inhibitors, J. Med. Chem. 36, S.314-319 (1993)]. Die interessanteste Verbindung war D-1-Tiq-Pro-Arg-H, die verglichen mit Boc-D-Phe-Pro-Arg-H, den doppelten Wirksamkeitsgewinn ergab. Jedoch wird Trypsin in gleichem Maße wie das Thrombin gehemmt.

Aus den heute zugänglichen Daten ist klar, daß, obgleich es einige effektive antigerinnungswirksame Verbindungen gibt, ein Bedarf an leistungsfähigen Antithrombinmitteln besteht, die schnell wirken, um die Pfropfenbildung zu verhindern und die nicht mit anderen Proteaseaktivitäten, z.B. der Plasminwirkung beim Auflösen des Pfropfens interferieren.

Ausgehend von diesem Stand der Technik lag der vorliegenden Erfindung die Aufgabe zugrunde, Verbindungen bereitzustellen, die im Sinne einer Thrombinhemmung biologisch aktiv sind und die Nachteile des vorhergehend beschriebenen Standes der Technik vermeiden. Das der Erfindung zugrundeliegende Problem bestand darüberhinaus darin, Thrombin spezifisch mit geringen Wirkstoffkonzentrationen und geringer Zelltoxizität zu inhibieren.

Erfindungsgemäß wird diese Aufgabe durch die Verbindung mit der Formel

Y¹-(NH-X¹-C=O)-(NH-X²-C=O)-(NH-X³-C=O)-(NH-X³-C=O)-(NH-X⁵-C=O)-(NH-X⁶-C=O)-Y² (I)

gelöst,
wobei Y¹
Wasserstoff
ist,
wobei (NH-X¹-C=O)
1. Tranexamic acid (=Trans-4-(Aminomethyl)cyclohexancarbonsäure) oder
2. Trans-4-(Guanidinomethyl)cyclohexancarbonsäure ist,
wobei (NH-X²-C=O)
durch eine chemische Bindung ersetzt wird,
wobei (NH-X³-C=O)
D-Cyclohexylalanin
ist,
wobei (NH-X⁴-C=O)
L-Azetidin-2-carbonsäure
ist,
wobei (NH-X⁵-C=O)
D-Tyrosin
ist,
wobei (NH-X⁶-C=O)
L-Homoarginin
ist,
wobei Y²
eine Aminogruppe (bei der C-endständigen Aminosäure ist die Carbonsäure- durch eine Amid-Gruppe ersetzt) ist.

Die erfindungsgemäßen Verbindungen können zur Hemmung aller Thrombin-vermitselten oder Thrombin-assozüerten Funktionen und Prozesse verwendet werden. Pharmazeutische Zusammenstellungen, die diese Verbindungen enthalten, wie auch Methoden zur Behandlung und Prophylaxe von Gefäßerkranküngen, entzündlichen Reaktionen, Karzinomen und neurodegenerativen Erkrankungen, die diese Verbindungen verwenden, sind ebenfalls Gegenstand der Erfindung. Die Verbindungen können auch zur *ex vivo* Darstellung verwendet werden, zur Lagerung und Behandlung von Blut außerhalb des Körpers und zur Beschichtung von invasiven Geräten. Ferner können die Verbindungen einem Patienten (unter Patient wird hier ein Mensch oder Tier verstanden) in Kombination mit einem fibrinolytischem Mittel verabreicht werden, um die Wirksamkeit einer gegebenen Dosis zu erhöhen oder die zur Erzielung eines gewünschten Effekts notwendige Dosis zu verringern, wie das Auflösen und Blutpfropfens oder die Verhinderung der Wiederverstopfung des zuvor blockierten Blutgefäßes.

Aufgrund ihres hohen Potentials und der Tatsache, daß sie durch chemische Synthesetechniken hergestellt werden können, können die Verbindungen preisgünstig in kommerziell praktikablen Mengen produziert werden. Die Peptide werden in passende Salzformen wie Acetate und Sulfate umgewandelt.

Darüber hinaus sind die erfindungsgemäßen Moleküle wesentlich kleiner als Hirudin und die anderen bisher beschriebenen peptidischen Thrombinhemmer. Daher ist es unwahrscheinlicher, eine unerwünschte Reaktion des Immunsystems bei mit diesen Substanzen behandelten Patienten hervorzurufen. Dementsprechend ist der Einsatz dieser Thrombin-Hemmer nicht auf die Behandlung akuter Erkrankungen beschränkt. Diese Verbindungen können auch in der Therapie chronischer thrombo-embolitischer Erkrankungen wie der Arteriosklerose oder der Restenosis infolge einer Angioplastie, eingesetzt werden. Die erfindungsgemäßen Verbindungen können auch in einer Vielzahl anderer Anwendungen anstelle natürlichen und rekombinanten Thrombins eingesetzt werden.

Wie man aus der Offenlegung folgern kann, sind die erfindungsgemäßen Verbindungen, Zusammenstellungen und Verfahren nützlich zur Behandlung und Vorsorge verschiedener Krankheiten in Zusammenhang mit unerwünschten Wirkungen des Thrombins, wie auch für diagnostische Zwecke.

Schließlich soll erwähnt werden, daß die Moleküle dieser Erfindung als Leitstrukturen zur Entwicklung von Molekülen mit noch vorteilhafteren Eigenschaften in Hinblick auf die oben genannten Anwendungen dienen können.

Pharmazeutisch akzeptable Salze von Peptiden dieser Erfindung beinhalten die durch Säurezugabe erzeugten Salze, die aus anorganischen Säuren und Carbonsäuren gebildet werden. Die Verbindungen, die durch die Formel (I) repräsentiert werden, werden durch bekannte Methoden der Peptidkopplung hergestellt.

In einer bevorzugten Ausführungsform liegen die erfindungsgemäßen Verbindungen als Verbindungsgemisch vor, das durch seinen Gehalt an mindestens zwei der erfindungsgemäßen Verbindungen charakterisiert ist. Bevorzugte pharmazeutisch akzeptable Salze der Verbindungen werden mit einer anorganischen Säure gebildet. Besonders bevorzugt ist dabei die Bildung eines pharmazeutisch akzeptablen Salzes mit Chlorwasserstoffsäure, Chlorsäure, Bromwasserstoffsäure, Bromsäure und / oder einer anderen Halogensäure. Eine weitere besonders bevorzugte Ausführungsform besteht in der Bildung eines pharmazeutisch akzeptablen Salzes mit Schwefelsäure und / oder Phosphorsäure. Vorteilhaft kann auch ein pharmazeutisch akzeptables Salz mit einer organischen Säure gebildet werden. Besonders bevorzugt ist dabei die Bildung des pharmazeutisch akzeptablen Salzes mit Essigsäure, Propionsäure, Malonsäure, Maleinsäure, Zitronensäure, Bernsteinsäure, Fumarsäure, Äpfelsäure, Benzoesäure, und / oder einer ähnlichen Carbonsäuren. Die durch Säurezugabe gebildeten Salze werden auf konventionelle Weise hergestellt, z.B. durch Neutralisieren der freien Basenform der Verbindung (I) mit der Säure.

Die erfindungsgemäßen Substanzen können in Verbindungen und Methoden zur Hemmung aller Thrombin-vermittelten oder Thrombin-assozüerten Funktionen verwendet werden. Pharmazeutische Zusammensetzungen, die diese Moleküle enthalten, sind, wie auch Methoden zur Behandlung und Prophylaxe von Gefäßerkrankungen, entzündlichen Reaktionen, Karzinomen und neurodegenerativen Erkrankungen, die diese Verbindungen verwenden, ebenfalls Teil der Erfindung. Die Substanzen können auch in Zusammensetzung zur *ex vivo*-Darstellung verwendet werden, zur Lagerung und zur Behandlung von Blut außerhalb des Körpers und zur Beschichtung von invasiven Geräten. Ferner können die erfindungsgemäßen Verbindungen einem Patienten (unter Patient wird hier ein Mensch oder ein Tier verstanden) in Kombination mit einem fibrinolytischen Mittel verabreicht werden, um die Wirksamkeit einer gegebenen Dosis zu erhöhen oder um die Erzielung eines gewünschten Effektes, wie das Auflösen eines Blutpfropfens oder das Verhindern der Wiederverstopfung des zuvor blockierten Blutgefäßes notwendige Dosis zu verringern.

Aufgrund ihres hohen Potentials und der Tatsache, daß sie durch chemische Synthesetechniken hergestellt werden können, sind die erfindungsgemäßen Substanzen preisgünstig in kommerziell praktikabler Menge produzierbar. Bevorzugt werden die Peptide in passende Salzformen, wie Acetate oder Sulfate umgewandelt.

Die Erfindung betrifft auch ein Arzneimittel, das durch seinen Gehalt an einer oder mehreren erfindungsgemäßen Verbindungen mit den üblichen Trägerstoffen, Hilfsmitteln oder Zusatzstoffen charakterisiert ist. Ferner ist auch eine Diagnostikzusammensetzung mit einem Gehalt an einer oder mehreren der Verbindungen Gegenstand der Erfindung.

Ein weiterer Gegenstand der Erfindung besteht in der Verwendung der Verbindung als Thrombininhibitor sowie zur Herstellung eines Arzneimittels zur Thrombininhibierung, Inhibierung der Fibrinbildung und / oder zur Inhibierung der Bildung eines Gerinnungspfropfens.

Die Verwendung einer oder mehrerer der Verbindungen zur Herstellung einer diagnostischen Zusammensetzung ist ebenfalls Gegenstand der Erfindung.

Die erfindungsgemäßen Verbindungen weisen gegenüber bisher bekannten Thrombininhibitoren vielfältige Vorteile auf. Insbesondere sind die Peptide leicht synthetisierbar, bereits wenig modifiziert wirksam und weisen eine hohe Wirksamkeit bei gleichzeitig hoher Spezifität und geringer Toxizität auf. Darüber hinaus dienen die kleinen Peptide, anders als Hirudin und Hirolog als Leitstrukturen für vorzugsweise oral verfügbare Wirkstoffe.

Die nachfolgenden Beispiele sollen die Erfindung näher erläutern, ohne diese einzuschränken.

### Beispiel 1 (nicht erfindungsgemäß)

### N-Acetyl-D-Gln-D-His-L-cha-D-Pro-D-Tyr-L-Arg-amid SEQ ID No. 1

Dieses Peptid wurde durch eine Festphasen-Synthese mithilfe eines ABIMED-Synthesizers AMS 96 (ABIMED Analysen-Technik GmbH, Langenfeld, Deutschland) hergestellt. Im Detail ließ man 1 meq Rink Amid-Harz sequentiell mit 2 x 5 meq geschützter Aminosäure reagieren. Die Aktivierung erfolgte mit 2 x 5 meq TBTU (O-benzotriazol-1-yl)-N, N, N' ,N' -tetramethyluroniumtetrafluoroborat. Nach bis zu 6 Zyklen der Synthese wurde der N-Terminus mit Essigsäureanhydrid acetyliert. Dann wurde der Schutz des Peptids durch eine Behandlung mit 90% TFA, 2,5% Trüsopropylsilan, 2,5% H₂O und 5% Dichlormethan aüfgehoben. Im selben Schritt erfolgte die Entkopplung des Peptids von seinem Träger. Die Testverbindung wurde anschließend an einen Trocknungsschritt in 20 µl Trifluoressigsäure angelöst, und dann mit 2x750 µl kaltem Butylether bei - 20 °Celsius inkubiert. Nach Zentrifugation wurde der Überstand abgenommen und der Rest Ether verdunstet. Die Identität der Produkte wurde stichprobenartig durch Massenspektroskopie bestätigt.

Die Hemmung des Thrombins wurde durch in-vitro-Hemmung der Amidase-Aktivität des Thrombins bestimmt und beträgt bei einer Peptidkonzentration von 1 µM 59%. Die Werte der hemmenden Konstante Kᵢ wurden aus Assays gewonnen, in denen Thrombin das fluorogene Substrat Tos-Gly Pro-Arg (7-amino-4-Methylcumarin) hydrolisiert. Die Assays wurde in 30 µl Assaypuffer (0,05 M Tris, 0,1 M NaCl, 0,1% PEG 8000, pH 7,6) mit 10 µl humaner Thrombinlösung (10⁻⁵ U/µl im Assaypuffer) und 140 µl einer Lösung des fluorogenen Substrats in einem Assaypuffer bei einer Konzentration von 30 µM durchgeführt. Lösungen der Testverbindung (10 µl) wurden bei verschiedenen Konzentrationen hinzugefügt. Die Raten der Hydrolyse des Substrats wurden durch Überwachung der Reaktionen bei 460 nm der Freisetzung von 7-Amino-4-Methylcumarin unter Verwendung von AMC gemessen. Die Realtion erreichte einen Gleichgewichtszustand innerhalb von drei Minuten, nachdem Thrombin mit dem Substrat und einem Hemmer vermischt wurden. Die kinetische Daten der konkurrierenden Hemmung (Kₘ, Vₘₐₓ und Kᵢ) wurden mittels der Darstellung nach Hanes analysiert (A/V gegen A bei verschiedenen Werten von A, dabei ist A die Substratkonzentration und V die Reaktionsgeschwindigkeit).

### Beispiel 2 (nicht erfindungsgemäß)

### N-Acetyl-D-Glu-L-Cha-D-Pro-D-Tyr-L-Arg-amid SEQ ID No. 2

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausfunrungsbeispiel 1 bestimmt und beträgt bei einer Peptidkonzentration von 1 µM 58%.

### Beispiel 3 (nicht erfindungsgemäß)

### N-Acetyl-D-Val-D-His-L-Cha D-Pro-D-Tyr-L-Arg-amid SEQ ID No. 3

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 1 µM 51% .

### Beispiel 4 (nicht erfindungsgemäβ)

### N-Acetyl-L-Ala-L-Cha-D-Pro-D- Tyr-L-Arg-amid SEQ ID No. 4

Dieses Peptid wurde wie in Beispiel beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 1 µM 44%.

### Beispiel 5 (nicht erfindungsgemäß)

### N-Acetyl-L-Ile-L-Arg-L-Cha-D-Pro-D-Tyr-L-Arg-amid SEQ ID No. 5

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 1 µM 61%.

### Beispiel 6 (nicht erfindungsgemäß)

### N-Acetyl-L-Tyr-L-Cit-L-Cha-D-Pro-D-Tyr-L-Arg-amid SEQ ID No. 6

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 1 µM 55%.

### Beispiel 7 (nicht erfindungsgemäß)

### N-Ar-etyl-L-Ser-L-Ser-L-Cha-D-Pro-D-Tyr-L-Arg-amid SEQ ID No. 7

Dieses Peptid wurde wie in Beispiel 1 beschrieben synthetisiert und für den Einsatz im Assay vorbereitet.

Die Hemmung des thrombins wurde durch in-vitro-Hemmung der Amidase-Aktivität des Thrombins wie in Ausführungsbeispiel 1 bestimmt und beträgt bei einer Peptidkonzentration von 1 µM 40%.

### Beispiel 8 (nicht erfindungsgemäß)

### N-Acetyl-D-Val-L-Cha-D-Pro-D-Tyr-L-Arg-amid SEQ ID No. 8

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 1 µM 45%.

### Beispiel 9 (nicht erfindungsgemäß)

### N-Acetyl-L-Trp-L-Cha-D-Pro-D-Tyr-L-Arg-amid SEQ ID No. 9

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 1 µM 63%.

### Beispiel 10 (nicht erfindungsgemäß)

### N Acetyl-L-Ser-L-Ala-L-Cha-D-Pro-D-Tyr-L-Arg-amid SEQ ID No. 10

Dieses Peptid wurde wie in Beispiel 1 beschrieben synthetisiert und für den Einsatz im Assay vorbereitet.

Die Hemmung des Thrombins, wurde durch in-vitro-Hemmung der Amidase-Aktivität des Thrombins wie in Ausführungsbeispiel 1 bestimmt und beträgt bei einer Peptidkonzentration von 1 µM 47%.

### Beispiel 11 (nicht erfindungsgemäß)

### N-Acetyl-L-Ser-L-Arg-L-Cha-D-Pro-D-Tyr-L-Arg-amid SEQ ID No. 11

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausführgsbeispiel 1 bestimmt und beträgt bei einer Peptidkonzentration von 1 µM 51%.

### Beispiel 12 (nicht erfindungsgemäß)

### N-Acetyl-D-Lys-L-Nle-L-Cha-D-Pro-D-Tyr-L-Arg-amid SEQ ID No. 12

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 1 µM 48%.

### Beispiel 13 (nicht erfindungsgemäß)

### N-Acetyl-D-Tyr-L-Cha-D-Pro-D-Tyr-L-Arg-amid SEQ ID No. 13

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 1 µM 47%.

### Beispiel 14 (nicht erfindungsgemäß)

### N-Acetyl->Arg-L-Cha-D-Pro-D-Tyr-Arg-amid SEQ ID No. 14

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 1 µM 47%.

### Beispiel 15 (nicht erfindungsgemäß)

### N-Acetyl-L-Tyr-D-Pro-L-Cha-D-Pro-D-Tyr-L-Arg-amid SEQ ID No. 15

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 1 µM 44%.

### Beispiel 16 (nicht erfindungsgemäß)

### N-Acetyl-L-Ala-D-Cha-L-Aze-D-Tyr-L-Arg-amid SEQ ID No. 16

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 1 µM 63%.

### Beispiel 17 (nicht erfindungsgemäß)

### N-Acetyl-L-Ala-D-Cha-L-Pro-D-Tyr-L-Har-amid SEQ ID No. 17

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 1 µM 56%.

### Beispiel 18 (nicht erfindungsgemäß)

### N-Acetyl-L-Trp-D-Cha-L-Aze-D-Tyr-L-Har-amid SEQ ID No. 18

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 250 nM 76%.

### Beispiel 19 (nicht erfindungsgemäß)

### N-Acetyl-L-Ala-D-Cha-L-Aze-D-Tyr-L-Har-amid SEQ ID No. 19

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 250 nM 77% .

### Beispiel 20 (nicht erfindungsgemäß)

### N-Acetyl-D-Phe-D-Cha-L-Aze-D-Tyr-L-Har-amid SEQ ID No. 20

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausnihrungsbeispiel 1 bestimmt und beträgt bei einer Peptidkonzentration von 250 nM 77% .

### Beispiel 21 (nicht erfindungsgemäß)

### N-Acetyl-L-Dcp-D-Cha-L-Aze-D-Tyr-L-Har-amid SEQ ID No. 21

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 250 nM 75%.

### Beispiel 22 (nicht erfindungsgemäß)

### N-Acetyl-L-Nhm-D-Cha-L-Aze-D-Tyr-L-Har-amid SEQ ID No. 22

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 250 nM 80%.

### Beispiel 23 (nicht erfindungsgemäß)

### N-Acetyl-L-Iq3-D-Cha-L-Aze-D-Tyr-L-Har-amid SEQ ID No. 23

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel bestimmt und beträgt bei einer Peptidkonzentration von 250 nM 72%.

### Beispiel 24 (nicht erfindungsgemäß)

### N-Aceiyl-L-Ppd-D-Cha-L-Aze-D-Tyr-L-Har-amid SEQ ID No. 24

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 250 nM 76%.

### Beispiel 25 (nicht erfindungsgemäß)

### N-Acetyl-L-Tea-D-Cha-L-Aze-D-Tyr-L-Har-amid SEQ ID No. 25

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 250 nM 74%.

### Beispiel 26 (nicht erfindungsgemäß)

### N-Acetyl-L-Phg-D-Cha-L-Aze-D-Tyr-L-Har-amid SEQ ID No. 26

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausfühnmgsbeispiel 1 bestimmt und beträgt bei einer Peptidkonzentration von 250 nM 95% .

### Beispiel 27 (nicht erfindungsgemäß)

### N-Acetyl-L-Nle-D-Cha-L-Aze-D-Tyr-L-Har-amid SEQ ID No. 27

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 250 nM 89%.

### Beispiel 28 (nicht erfindungsgemäß)

### N-Acetyl-Lcha-D-Cha-L-Aze-D-Tyr-L-Har-amid SEQ ID No. 28

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 250 nM 90%.

### Beispiel 23 (nicht erfindungsgemäß)

### N-Acetyl-L-Pnp-D-Cha-L-Aze-D-Tyr-L-Har-amid SEQ ID No. 29

Dieses Peptid wurde wie in Beispiel 1 beschrieben synthetisiert und für den Einsatz im Assay vorbereitet.

Die Hemmung des Thrombins wurde durch in-vitro-Hemmung der Amidase-Aktivität des Thrombins wie in Ausführungsbeispiel 1 bestimmt und beträgt bei einer Peptidkonzentration von 250 nM 72%.

### Beispiel 30

### Tranexamic acid-D-Cha-L-Aze-D-Tyr-L-Har-amid SEQ ID No. 30

Dieses Peptid wurde wie in Beispiel 1 beschrieben synthetisiert und für den Einsatz im Assay vorbereitet.

Für die Hemmung des Thrombins wurde ein Kᵢ von 7nM bestimmt.

### Beispiel 31

### Trans-4-(Guanidinomethyl)cyclohexancarbonsäure-D-Cha-L-Aze-D-Tyr-L-Har-amid SEQ ID No. 31

Dieses Peptid wurde wie in Beispiel 1 beschrieben synthetisiert und für den Einsatz im Assay vorbereitet.

Für die Hemmung des Thrombins wurde ein K*ᵢ* von 3nM bestimmt.
1. Verbindung, gekennzeichnet, durch die Zusammensetzung um Tranexamic acid-D-Cha-L-Aze-D-Tyr-L-Har-amid (SEQ ID NO. 30) und pharmazeutisch akzeptable Salze davon.
2. Verbindung, gekennzeichnet durch die Zusammensetzung um Trans-4-(Guanidinomethyl)cyclohexancarbonsäure-D-Cha-L-Aze-D-Tyr-L-Har-amid (SEQ ID NO. 31) und pharmazeutisch akzeptable Salze davon.
3. Verbindungsgemisch, gekennzeichnet durch einen Gehalt an mindestens zwei Verbindungen nach mindestens einem der Ausführungsformen 1 bis 2.
4. Verbindung nach einer der Ausführungsformen 1 bis 3, wobei die Verbindung als pharmazeutisch akzeptables Salz vorliegt, das mit einer anorganischen säure gebildet wird.
5. Verbindung nach Ausführungsform 4, wobei die Verbindung als pharmazeutisch akzeptables Salz vorliegt, das mit Chlorwasserstoffsäure, Bromsäure und / oder einer anderen Halogensäure gebildet wird.
6. Verbindung nach Ausfiihrungsform 4, wobei die Verbindung als pharmazeutisch akzeptables Salz vorliegt, das mit Schwefelsäure und / oder Phosphorsäure gebildet wird.
7. Verbindung nach mindestens einer der Ausführungsformen 1 bis 3, wobei die Verbindung als pharmazeutisch akzeptables Salz vorliegt, das mit einer organische Säure gebildet wird.
8. Verbindung nach Ausführungsform 7, wobei die Verbindung als pharmazeutisch akzeptables Salz vorliegt, das mit Essigsäure, Propionsäure, Malonsäure, Maleinsäure, Zitronensäure, Bernsteinsäure, Malsäure, Benzoesäure, Fumarsäure und / oder einer ähnlichen Carbonsäure gebildet wird.
9. Arzneimittel, gekennzeichnet durch seinen Gehalt an einer oder mehreren Verbindungen nach mindestens einem der Ausführungsformen 1 bis 8 neben üblichen Trägerstoffen, Hilfsmitteln und / oder Zusatzstoffen.
10. Diagnostische Zusammensetzung, gekennzeichnet durch ihren Gehalt an einer oder mehreren Verbindungen nach mindestens einem der Ausführungsformen 1 bis 8.
11. Verwendung einer oderer mehrerer Verbindungen nach mindestens einem der Ausführungsformen 1 bis 8 zur Herstellung eines Arzneimittels zur Thrombinhemmung, Inhibierung der Fibrinbildung und / oder zur Inhibierung der Bildung eins Gerinnungspfropfens.
12. Verwendung einer oder mehrerer Verbindungen nach mindestens einem der Ausführungsformen 1 bis 8 zur Herstellung einer diagnostischen Zusammensetzung.
13. Eine pharmazeutische Zusammenstellung, die eine wirkungsvolle Pfropfen-verhindernde Menge einer Verbindung aus Ausführungsform 1 bis 8 und einem pharmazeutisch akzeptablen Träger umfaßt. Die Verbindungen können auch das Substrat sein.

### Beschreibung der Abkürzungen

- Ala: = Alanin
- Val: = Valin
- Leu: = Leucin
- De: = Isoleucin
- Pro: = Prolin
- Phe: = Phenylalanin
- Phg: = Phenylglycin
- Cha: = Cyclohexylalanin
- Trp: = Tryptophan
- Met: = Methionin
- Gly: = Glycin
- Ser: = Serin
- Thr: = Threonin
- Cys: = Cystein
- Tyr: = Tyrosin
- Asn: = Asparagin
- Gln: = Glutamin
- Asp: = Asparaginsäure
- Glu: = Glutaminsäure
- Lys: = Lysin
- Arg =: Arginin
- His: = Histidin
- Nle: = Norleucin
- Orn: = Ornithin
- Cit: = Citrullin
- Aze: = Azetidin
- Har: = Homoarginin
- Dcp: = Dichlorphenylalanin
- Nhm: = Tetrahydronorhaman-3-carboxylsäule
- Iq3: = Tetrahydroisoquinolin-(1,2,3,4)-3-carboxylsäure
- Ppd: = 4-Phenylpiperidin-4-carbonsäure
- Tea: = Thienylalanin
- Pnp: = Paranitrophenylalanin

## Patentansprüche

1. Tranexamsäure -D-Cha-L-Aze-D-Tyr-L-Har-amid

2. Trans-4-(Guanidomethyl)cyclohexancarbonsäure-D-Cha-L-Aze-D-TYr-L-Har-amid

## Claims

1. Tranexamic acid-D-Cha-L-Aze-D-Tyr-L-Har amide.

2. Trans-4-(guanidinomethyl)cyclohexanecarboxylic acid-D-Cha-L-Aze-D-Tyr-L-Har amide.

## Revendications

1. D-Cha-L-Aze-D-Tyr-L-Har-amide d'acide tranexamique.

2. D-Cha-L-Aze-D-Tyr-L-Har-amide d'acide trans-4-(guanidinométhyl)cyclohexanecarboxylique.
